(19) Europäisches Patentamt / European Patent Office / Office européen des brevets

(11) **EP 4 030 157 B1**

(12) **EUROPEAN PATENT SPECIFICATION**

(45) Date of publication and mention of the grant of the patent:
**15.04.2026 Bulletin 2026/16**

(21) Application number: **20863632.4**

(22) Date of filing: **10.09.2020**

(51) International Patent Classification (IPC):
*G01K 13/20* (2021.01)      *A61B 5/01* (2006.01)
*A61B 5/02* (2006.01)      *A61B 5/0245* (2006.01)
*A61B 5/318* (2021.01)      *G01K 7/42* (2006.01)

(52) Cooperative Patent Classification (CPC):
**G01K 13/20; A61B 5/01; A61B 5/0245; G01K 7/427**

(86) International application number:
**PCT/JP2020/034305**

(87) International publication number:
**WO 2021/049573 (18.03.2021 Gazette 2021/11)**

(54) **CORE BODY TEMPERATURE ESTIMATING DEVICE, CORE BODY TEMPERATURE ESTIMATING METHOD, AND CORE BODY TEMPERATURE ESTIMATING PROGRAM**

VORRICHTUNG ZUR SCHÄTZUNG DER KÖRPERKERNTEMPERATUR, VERFAHREN ZUR SCHÄTZUNG DER KÖRPERKERNTEMPERATUR UND PROGRAMM ZUR SCHÄTZUNG DER KÖRPERKERNTEMPERATUR

DISPOSITIF D'ESTIMATION DE TEMPÉRATURE CORPORELLE CENTRALE, PROCÉDÉ D'ESTIMATION DE TEMPÉRATURE CORPORELLE CENTRALE ET PROGRAMME D'ESTIMATION DE TEMPÉRATURE CORPORELLE CENTRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2019 JP 2019164711**

(43) Date of publication of application:
**20.07.2022 Bulletin 2022/29**

(73) Proprietors:
• **University of Occupational and Environmental Health, Japan**
  **Kitakyushu-shi,**
  **Fukuoka 807-8555 (JP)**
• **Maeda Corporation**
  **Chiyoda-ku,**
  **Tokyo 1028151 (JP)**
• **Mitsufuji Corporation**
  **Kyoto, 619-0237 (JP)**

(72) Inventors:
• **KUROSAKA, Chie**
  **Kitakyushu-shi, Fukuoka 807-8555 (JP)**
• **SAKAMOTO, Hiroto**
  **Tokyo 102-8151 (JP)**
• **MATSUMOTO, Takeshi**
  **Kyoto 6190237 (JP)**

(74) Representative: **Novagraaf International SA**
**Chemin de l'Echo 3**
**1213 Onex, Geneva (CH)**

(56) References cited:
WO-A1-2017/213011      JP-A- 2016 150 084
JP-A- 2019 071 966      US-A1- 2001 008 954
US-A1- 2007 239 038      US-A1- 2010 280 331
US-A1- 2014 180 027      US-A1- 2014 180 027
US-A1- 2016 367 157      US-A1- 2018 220 901

EP 4 030 157 B1

**Description**

Technical Field

The present invention relates to a core body temperature estimation device, a core body temperature estimation method, and a core body temperature estimation program.

Background Art

In recent years, the tendency toward high temperature has become remarkable and the risk of heatstroke has increased. Accordingly, various techniques for heatstroke countermeasures have been disclosed (see, for example, PTL 1 and PTL 2). The technique described in PTL 1 relates to air-conditioned clothes having a body temperature monitoring function for the purpose of comprehensively managing the physical conditions of a worker, in addition to preventing an increase in body temperature in a hot environment.

The air-conditioned clothes having the body temperature monitoring function include a clothing portion worn by the worker, an air blowing device that blows air to the space between the clothing portion and the body of the worker, a body temperature measurement device that measures the body temperature of the worker wearing the clothing portion, and an air blowing control device that controls the driving of the air blowing device, based on the measurement result of the body temperature by the body temperature measurement device. When the measured body temperature is lower than a predetermined temperature, the air blowing control device stops air blowing by the air blowing device or reduces the amount of air blown. When the measured body temperature is higher than the predetermined temperature, the air blowing control device increases the amount of air blown by the air blowing device. A heart rate measurement device for measuring the heart rate of the worker is further included.

The technique described in PTL 2 detects the risk of heatstroke of a worker before an abnormality occurs in the core body temperature of the worker. This technique includes workload information input means for inputting the workload information of a worker, a respiration sensor for detecting the respiration of the worker in real time, an electrocardiograph for detecting the heart rate of the worker in real time, information recording means for recording respiration curve information acquired by the respiration sensor and heart rate information acquired by the electrocardiograph, with the respiration curve information being in sync with the heart rate information, RSA calculation means for deriving respiratory sinus arrhythmia (RSA), based on the respiration curve information and the heart rate information recorded in the information recording means, and display means for displaying an RSA calculation value calculate by the RSA calculation means and the workload information. Further prior art document US 2014/0180027 A1 discloses a method for determining the core body temperature of a person. An initial core body temperature is set and a heart rate of the person is received in a processor. A predicted core body temperature is then calculated using an extended Kalman filter, based on heart rate and initial core body temperature.

[Citation List]

[Patent Literature]

[PTL 1] Japanese Patent Application Publication No. 2017-166075
[PTL 2] Japanese Patent Application Publication No. 2017-27123

Summary of Invention

[Technical Problem]

Heatstroke is considered to be associated with core body temperature. Accordingly, the core body temperature of a worker is desirably measured to determine the risk of heatstroke of the worker. However, it is difficult to measure the core body temperature of the worker during labor work. Therefore, the core body temperature needs to be estimated based on physiological indexes that can be measured more easily than the core body temperature.

An object of the present invention is to provide a technique for estimating a core body temperature, based on physiological indexes.

[Solution to Problem]

The following means are adopted to solve the problems described above.

That is, according to the invention, there is provided a core body temperature estimation device as defined in claim 1.

Further according to the invention, a method as defined in claim 4 and a program as defined in claim 5 are provided.

[Advantageous Effects of Invention]

According to the present invention, it is possible to provide a technique for estimating core body temperature based on physiological indexes.

Brief Description of Drawings

Fig. 1 is a figure illustrating an example of the functional block of a core body temperature estimation model building device.

Fig. 2 is a figure illustrating an example of the functional block of a core body temperature estimation device.

Fig. 3 is a figure illustrating an example of the hardware structure of an information processing device.

Fig. 4 is a figure illustrating an example of an operation flow of building a core body temperature estimation model by the core body temperature estimation model building device.

Fig. 5 illustrates an example of a Poincaré plot of an RRI.

Fig. 6 is a figure illustrating an example of the relationship between the estimated value TMP of the core body temperature and the change amount ΔTMP of the core body temperature.

Fig. 7 is a figure illustrating an example of an operation flow of the estimation of the core body temperature by the core body temperature estimation device.

Fig. 8 is a graph illustrating an example of changes in the core body temperature actually measured during an exercise stress test and the estimated core body temperature based on heart rate information when the exercise stress test is performed on a subject A.

Fig. 9 is a graph illustrating an example of changes in the core body temperature actually measured during an exercise stress test and the estimated core body temperature based on heart rate information when the exercise stress test is performed on a subject B.

Fig. 10 is a graph illustrating an example of changes in the core body temperature actually measured during an exercise stress test and the estimated core body temperature based on heart rate information when the exercise stress test is performed on a subject C.

Fig. 11 is a graph illustrating an example of changes in the core body temperature actually measured during an exercise stress test and the estimated core body temperature based on heart rate information when the exercise stress test is performed on a subject D.

Fig. 12 is a graph illustrating an example of changes in the core body temperature actually measured during an exercise stress test and an estimated core body temperature based on heart rate information when the exercise stress test is performed on a subject E.

Description of Embodiments

An embodiment will be described below with reference to the drawings. The structure of the embodiment is an example and the structure of the invention is not limited to the specific structure of the disclosed embodiment. In carrying out the invention, the specific structure according to an embodiment may be adopted as appropriate.

[Embodiment]

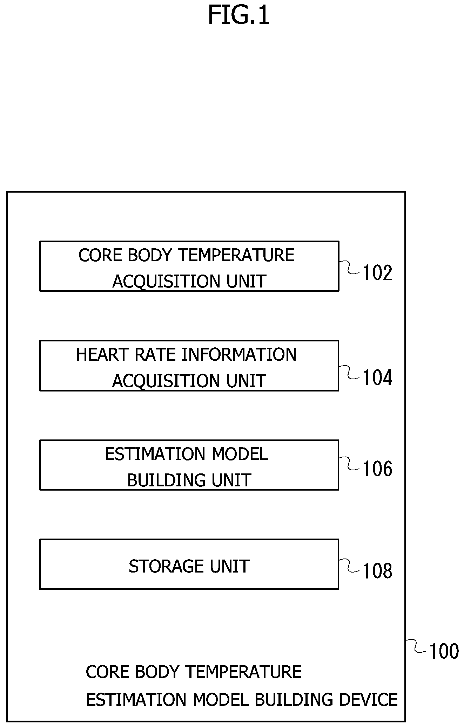

Fig. 1 is a figure illustrating an example of the functional block of a core body temperature estimation model building device according to the embodiment. A core body temperature estimation model building device 100 in Fig. 1 includes a core body temperature acquisition unit 102, a heart rate information acquisition unit 104, an estimation model building unit 106, and a storage unit 108.

The core body temperature estimation model building device 100 acquires the core body temperature and heart rate information when a plurality of model building subjects are working under predetermined stresses and builds a core body temperature estimation model for estimating the core body temperature.

The core body temperature acquisition unit 102 acquires the core body temperature of each of the model building subjects measured by thermometer or the like for measuring the core body temperature. The core body temperature is, for example, rectal temperature.

The heart rate information acquisition unit 104 acquires the heart rate information of each of the model building subjects during work measured by an electrocardiograph or the like for measuring the heart rate information. The heart rate information is, for example, electrocardiogram data or heart rate data. The electrocardiograph is an example of a pulsometer that measures beats.

The estimation model building unit 106 builds a core body temperature estimation model that estimates the core body temperature by a regression analysis based on the core body temperature after the initial time and the heart rate information after the initial time of each of the model building subjects.

The storage unit 108 stores programs, models, data, and the like used in the core body temperature estimation model building device 100. The storage unit 108 stores the built core body temperature estimation model, the core body temperature, the heart rate information, and the like.

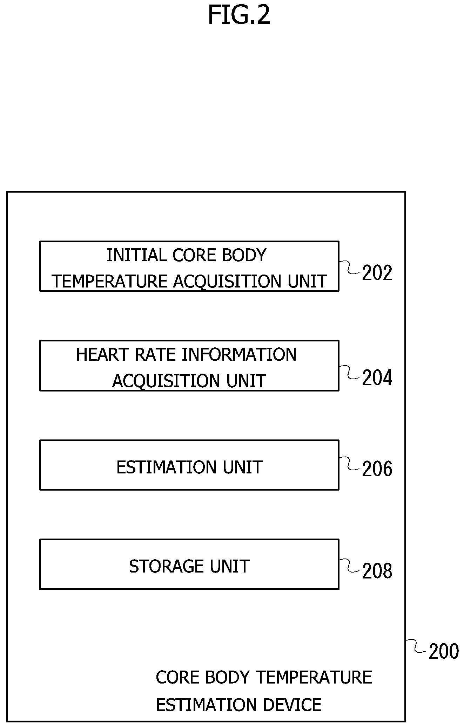

Fig. 2 is a figure illustrating an example of the functional block of a core body temperature estimation device according to the embodiment. A core body temperature estimation device 200 in Fig. 2 includes an initial core body temperature acquisition unit 202, a heart rate information acquisition unit 204, an estimation unit 206, and a storage unit 208.

The core body temperature estimation device 200 acquires the core body temperature, at the initial time, of the estimation target subject targeted for core body temperature estimation and the heart rate information of the estimation target subject during work, and estimates the core body temperature of the estimation target subject using the core body temperature estimation model built by the core body temperature estimation model building device 100. The model building subjects and the estimation target subject are also collectively referred to simply as the subjects.

The initial core body temperature acquisition unit 202 acquires the core body temperature of the estimation target subject before work measured by a thermometer or the like for measuring the core body temperature.

The heart rate information acquisition unit 204 acquires the heart rate information of the estimation target subject during work measured by an electrocardiograph or the like for measuring the heart rate information. The heart rate information is, for example, electrocardiogram data or heart beat data. The heart rate information acquisition unit 204 is an example of the beat acquisition unit.

The estimation unit 206 estimates the change amount of the core body temperature in each of the reference periods using the core body temperature estimation model built by the core body temperature estimation model building device 100 based on the core body temperature at the first time (arbitrary time) of the estimation target subject and the heart rate information in the period including the first time.

The storage unit 208 stores programs, models, data, and the like used in the core body temperature estimation device 200. The storage unit 208 stores the core body temperature estimation model built by the core body temperature estimation model building device 100, the estimated change amount of the core body temperature, the core body temperature, the heart rate information, and the like.

Although heart rate information is used here, other beat information may be used instead of heart rate information. A heart rate is the beat of a heart. A heart rate is one type of a beat. A beat is a motion that occurs when an internal organ is repeatedly contracted and relaxed. Other examples of a beat include a fingertip beat wave and an earlobe beat wave. A heart rate is the number of heart beats in a certain period. A pulse beat is the beat of an artery. A beat interval is one cycle of a beat.

The core body temperature estimation model building device 100 and the core body temperature estimation device 200 can be achieved by use of a dedicated or general-purpose computer such as a personal computer (PC), a work station (WS), a smartphone, a mobile phone, a tablet terminal, a car navigation device, and a personal digital assistant (PDA), or an electronic device having a computer.

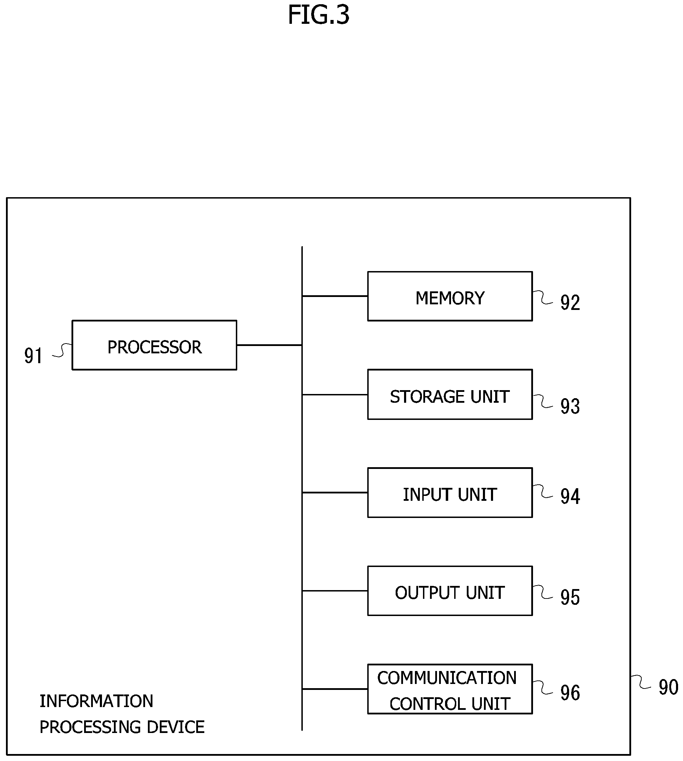

Fig. 3 is a figure illustrating an example of the hardware structure of an information processing device. The information processing device illustrated in Fig. 3 has the structure of a general computer. The core body temperature estimation model building device 100 and the core body temperature estimation device 200 are achieved by an information processing device 90 as illustrated in Fig. 3. The information processing device 90 in Fig. 3 includes a processor 91, a memory 92, a storage unit 93, an input unit 94, an output unit 95, and a communication control unit 96. These are connected to each other by a bus. The memory 92 and the storage unit 93 are computer-readable recording media. The hardware structure of the computer is not limited to the example illustrated in Fig. 3 and components may be omitted, replaced, or added as appropriate.

The information processing device 90 can achieve the function that meets a predetermined purpose by causing the processor 91 to load a program stored in the recording media into the work area of the memory 92 and execute the program and the components and the like to be controlled through the execution of the program.

The processor 91 is, for example, a central processing unit (CPU) or a digital signal processor (DSP).

The memory 92 includes, for example, a random access memory (RAM) and a read only memory (ROM). The memory 92 is also referred to as a main storage device.

The storage unit 93 is, for example, an erasable programmable ROM (EPROM) or a hard disk drive (HDD). In addition, the storage unit 93 may include a removable medium, that is, a portable recording medium. The removable medium is, for example, a universal serial bus (USB) memory or a disc recording medium such as a compact disc (CD) or a digital versatile disc (DVD). The storage unit 93 is also referred to as a secondary storage device.

The storage unit 93 stores various types of programs, various types of data, and various types of tables in the recording medium in a readable and writable manner. The storage unit 93 stores an operating system (OS), various types of programs, various types of tables, and the like. The information stored in the storage unit 93 may be stored in the memory 92. In addition, the information stored in the memory 92 may be stored in the storage unit 93.

The operating system is the software that mediates between software and hardware, manages memory space, manages files, manages processes and tasks, and so on. The operating system includes a communication interface. The communication interface is the program that exchanges data with other external devices and the like connected via the communication control unit 96. The external devices and the like include, for example, other computers, external storage devices, and the like.

The input unit 94 includes a keyboard, a pointing device, a wireless remote controller, a touch panel, and the like. In addition, the input unit 94 may include an input device for video or images such as a camera or an input device for audio such as a microphone.

The output unit 95 includes a display device such as a liquid crystal display (LCD), an EL (electroluminescence) panel, a cathode ray tube (CRT) display, a plasma display panel (PDP), and an output device such as a printer. In addition, the output unit 95 may include an output device for audio such as a speaker.

The communication control unit 96 is connected to another device and controls communication between the computer 90 and the other device. The communication control unit 96 is, for example, a local area network (LAN) interface board, a wireless communication circuit for wireless communication, and a communication circuit for wired communication. The LAN interface board and the wireless communication circuit are connected to a network such as the Internet.

The computer that achieves the core body temperature estimation model building device 100 achieves the functions of the core body temperature acquisition unit 102, the heart rate information acquisition unit 104, and the estimation model building unit 106 by causing the processor to load programs stored in an auxiliary storage device into the main storage device and execute the programs. On the other hand, the storage unit 108 is provided in the storage area of the main storage device or the auxiliary storage device.

The computer that achieves the core body temperature estimation device 200 achieves the functions of the initial core body temperature acquisition unit 202, the heart rate information acquisition unit 204, and the estimation unit 206 by causing the processor to load programs stored in the auxiliary storage device into the main storage device and execute the programs. On the other hand, the storage unit 208 is provided in the storage area of the main storage device or the auxiliary storage device.

<Building of the core body temperature estimation model>

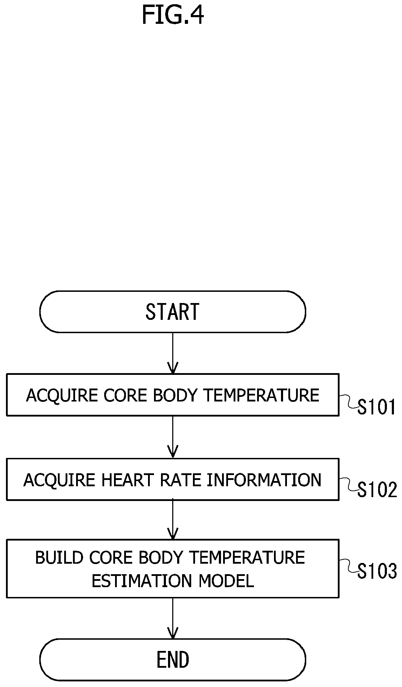

Fig. 4 is a figure illustrating an example of an operation flow of the building of a core body temperature estimation model by the core body temperature estimation model building device. Here, the core body temperature estimation model building device 100 acquires the core body temperature from the start time to the end time and the heart rate information from the start time to the end time of a predetermined period of a plurality of model building subjects when the plurality of model building subjects suffer a predetermined exercise stress test in the predetermined period, and builds a core body temperature estimation model for estimating the core body temperatures of the subjects. The core body temperature estimation model estimates the core body temperature for each of split reference periods resulting from dividing each having a predetermined time length (referred to as time P) from arbitrary first time based on, for example, the core body temperature at the first time and the heart rate information of the period including the first time. More specifically, for example, the core body temperature estimation model estimates the change amount of the core body temperature from time $t = nP$ to time $t = (n + 1)P$ based on the core body temperature at time $t = nP$ (which may be the estimated core body temperature) and the heart rate information from the time $t = (n - 1)P$ to the time $t = (n + 1)P$ (where n is an integer not less than 0). At the time of an exercise stress test, each of the model building subjects performs predetermined stress work under a predetermined environment (such as a predetermined temperatures and predetermined humidity) from the start time to the end time of a predetermined period. An exercise stress test is performed as follows. For example, the model building subjects rest for six minutes, suffer exercise stress for 18 minutes, rest for 18 minutes, suffer exercise stress for 24 minutes, and rest for 18 minutes in an artificial weather room in which, for example, the temperature is adjusted to 35°C and the humidity is adjusted to 50%. It is assumed that the exercise stress is applied by an ergometer and the stress value is 80 kW or 60% of the maximal oxygen uptake of each of the model building subjects measured in advance. During the exercise stress test, the core body temperature and the heart rate information of each of the model building subjects is measured continuously in parallel using a thermometer or the like and an electrocardiograph or the like, respectively. The exercise stress tests of the plurality of model building subjects may be performed concurrently in parallel or separately from each other.

In S101, the core body temperature acquisition unit 102 of the core body temperature estimation model building device 100 acquires the core body temperature of each of the model building subjects measured by a thermometer or the like for measuring the core body temperature. The core body temperature acquisition unit 102 acquires the core body temperature from the start time to the end time of the exercise stress test of each of the plurality of model building subjects through a clinical thermometer or the like connected to the core body temperature estimation model building device 100 directly or via a network or the like. In addition, the core body temperature acquisition unit 102 may cause the model building subjects or other managers to input the core body temperature through input means such as a keyboard so as to acquire the core body temperature from the start time to the end time of the exercise stress test. The core body temperature acquisition unit 102 stores the acquired core body temperatures in the storage unit 108.

In S102, the heart rate information acquisition unit 104 acquires the heart rate information from the start time to the end time of the exercise stress test of each of the plurality of model building subjects measured by an electrocardiograph or the like for measuring the heart rate information. The electrocardiograph is, for example, a device including electrodes attached to the skin of a model building subject and a transmission unit for storing and transmitting heart rate information measured by the electrodes. In addition, the electrocardiograph may be a wearable terminal including electrodes provided on a wear (such as a T-shirt) and a transmission unit for transmitting the electrocardiogram data measured by the electrodes. In addition, the electrocardiograph may also be a wearable terminal worn on a wrist for measurement such as a wristwatch or a wristband that acquires heart rate information, or a clip-type wearable terminal worn on a fingertip or an ear. The heart rate information acquisition unit 104 acquires the heart rate information from the start time to the end time of the exercise stress test of each of the model building subjects through an electrocardiograph or the like connected to the core body temperature estimation model building device 100 directly or via a network or the like. In addition, the heart rate information acquisition unit 104 may acquire the heart rate information via a network or the like from another information processing device or the like that has acquired the heart rate information from the electrocardiograph or the like. The heart rate information acquisition unit 104 acquires the heart rate information after the initial time. The heart rate information acquisition unit 104 stores the acquired heart rate information of the model building subjects in the storage unit 108.

The processing steps S101 and S102 may be exchanged with each other. In addition, the processing steps S101 and S102 may be performed in parallel. The core body temperature acquisition unit 102 and the heart rate information acquisition unit 104 may acquire the core body temperature and the heart rate information in parallel with the measurement of the core body temperature and the heart rate information during the exercise stress test of the model building subjects. The core body temperature and the heart rate information are stored in the storage unit 108 together with the time information indicating the measurement time.

In S103, the estimation model building unit 106 builds a core body temperature estimation model that estimates the core body temperature by a regression analysis based on the acquired core body temperature and heart rate information after the initial time of each of the model building subjects. For example, the core body temperature estimation model estimates the change amount of the core body temperature in each of the predetermined reference period at predetermined reference periods from the initial time. The core body temperature estimation model is built using, for example, the heart rate information and the core body temperature at the start of one reference period as explanatory variables and the change amount of the core body temperature in the reference period as an objective variable. The objective variable may be an estimated value of core body temperature. The core body temperature estimation model may be, for example, a model that estimates the core body temperature for each of the reference periods from the initial time.

The estimation model building unit 106 calculates the R-R interval (RRI) based on the acquired heart rate information. The RRI is the time difference between the time of occurrence of an R wave and the time of occurrence of the preceding R wave on an electrocardiogram. The RRI is has a length of one heart rate (one cycle). The RRI is an example of a beat interval. The variability in the RRI is referred to as heart rate variability (HRV). The estimation unit 106 calculates the RRI based on the acquired heart rate information. The estimation model building unit 106 divides the calculated RRI of each heart rate for each of the reference periods (for example, every three minutes) from the initial time and creates a Poincaré plot for each of the reference periods. The length of one heart rate calculated by another well-known method may be used instead of the RRI. The reference period is not limited to three minutes and may be longer or shorter than three minutes. The reference period is preferably one minute or more from a statistical viewpoint for calculating an index that reflects the heart rate variability, which will be described later. In addition, the reference period is preferably 15 minutes or shorter from the viewpoint of the accuracy of estimation of the core body temperature and the operation.

<<Example of a Poincaré plot>>

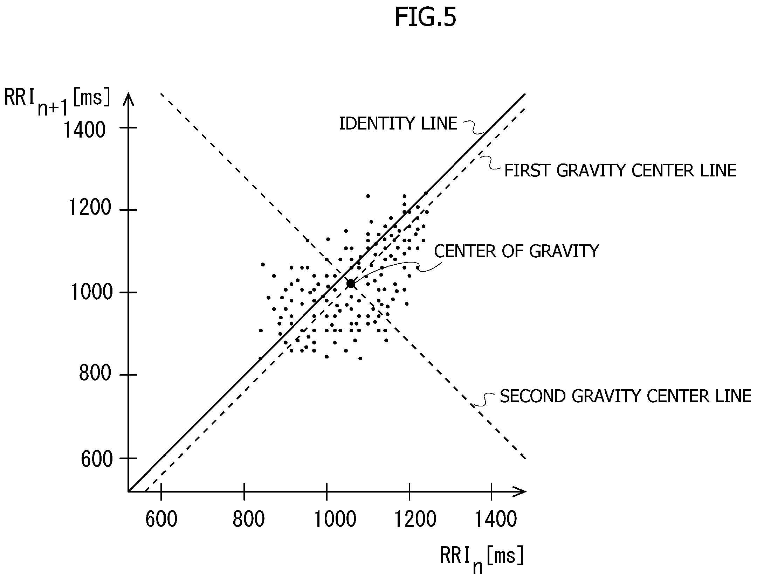

Fig. 5 illustrates an example of a Poincaré plot of the RRI. The Poincaré plot illustrated in Fig. 5 is an example of a Poincaré plot created for each of reference periods (for example, every three minutes) while a model building subject wears an electrocardiograph or the like for measuring heart rate information and performs predetermined work (exercise).

The Poincaré plot in Fig. 5 represents the relationship between the n-th beat and (n + 1)-th beat when the coordinate on the horizontal axis is the RRI (ms) of the n-th beat and the coordinate on the vertical axis is the RRI (ms) of the (n + 1)-th beat in one reference period. In addition, Fig. 5 represents the line in which the coordinate on the horizontal axis is identical to the coordinate on the vertical axis as an identity line. In addition, Fig. 5 represents the center of gravity of all plotted points. The coordinates (X, Y) of the center of gravity are acquired as (average value of the coordinates of the individual points on the horizontal axis, the average value of the coordinates of the individual points on the vertical axis). The straight line that passes through the center of gravity and is parallel to the identity line is referred to as the first gravity center line and the straight line that passes through the center of gravity and is orthogonal to the identity line is referred to as the second gravity center line. Here, it is assumed that the distance from the i-th point (coordinate on the horizontal axis: RRI of the i-th beat, coordinate on the vertical axis: RRI of the (i + 1)-th beat) to the identity line is $v_i$ and the distance from the i-th point to the second gravity center line is $h_i$. Using these, the indexes SD1 and SD2 based on the Poincaré plot of the RRI can be acquired as follows.

[Math. 1]

$$SD1^2 = \frac{1}{N} \sum_{i=1}^{N} v_i^2$$

$$SD2^2 = \frac{1}{N} \sum_{i=1}^{N} h_i^2$$

Where, N is the total number of points of one Poincaré plot. The indexes SD1 and SD2 reflect the heart rate variability. It should be noted that SD1 and SD2 are 0 or positive values. SD1 and SD2 are examples of indicators acquired by the analysis of the Poincaré plot of the RRI.

The estimation model building unit 106 calculates SD2 for each of the reference periods based on the RRI. SD2(x) represents SD2 of the x-th reference period (x-th reference period). The estimation unit 106 builds a core body temperature estimation model for estimating the core body temperature for each of reference periods (for example, every three minutes) from the initial time. Here, when the reference period is P, the time after a lapse of the first reference period from the initial time (t = 0) is t = 1 × P and the time after a lapse of the j-th reference period is t = jP. That is, the first reference period begins with the initial time (t = 0) and ends with the time (t = 1 × P) after a lapse of the first reference period. The j-th reference period begins with the time (t = (j - 1) × P) after a lapse of the (j - 1)-th reference period and ends with the time (t = j × P) after a lapse of the j-th reference period. The estimation model building unit 106 builds a core body temperature estimation model by performing a regression analysis that uses, as the explanatory variables, SD2(j) and SD2(j - 1) acquired by analysis of the Poincaré plot of the RRIs of the j-th reference period and the (j - 1)-th reference period and the estimated value (which is assumed to be TMP(j - 1)) of the core body temperature at time t = (j - 1)P after a lapse of the (j - 1)-th reference period (at the start of the j-th reference period) and uses, as the objective variable, the change amount (change amount of the core body temperature in the j-th reference period) of the core body temperature from time t = (j - 1)P to time t = jP. A statistical analysis method such as a multi-regression analysis or a logistic regression analysis can be used as the regression analysis. When the change amount (change amount of the core body temperature in the j-th reference period) of the core body temperature from time t = (j - 1)P to time t = jP is ΔTMP(j), ΔTMP(j) is expressed as follows.

[Math. 2]

$$\Delta TMP(j) = f\left(SD2(j), SD2(j-1), TMP(j-1)\right)$$

Where, f(SD2(j), SD2(j - 1), TMP(j - 1)) is a function of SD2(j), SD2(j - 1), and TMP(j - 1). In addition, TMP(0) is the core body temperature at the initial time. For example, f(SD2(j), SD2(j - 1), TMP(j - 1)) is expressed as follows.

[Math. 3]

$$f\left(SD2(j), SD2(j-1), TMP(j-1)\right)$$
$$= A1 + A2 \times \log SD2(j) + A3 \times SD2(j)/SD2(j-1) + A4 \times TMP(j-1)$$

Where, A1, A2, A3, and A4 are coefficients acquired by a regression analysis. In addition, log is common logarithm. The formula acquired by a regression analysis is not limited to the one illustrated here. In addition, the estimated value TMP(j) of the core body temperature at time t = jP is acquired as follows.

[Math. 4]

$$TMP(j) = TMP(0) + \sum_{i=1}^{j} \Delta TMP(i)$$

Where, TMP(0) is the core body temperature at the initial time (t = 0) acquired by measurement. The estimated value TMP(j) of the core body temperature at time t = jP is acquired by adding the change amount of the core body temperature in each of the reference periods from time t = 0 to time t = jP to the core body temperature TMP(0) at the initial time. The estimation model building unit 106 stores the built core body temperature estimation model ($\Delta$TMP, TMP) in the storage unit 108. As a result, the core body temperature estimation model is built by the core body temperature estimation model building device 100. In the core body temperature estimation model, ($\Delta$TMP, TMP) is expressed as a function of TMP(0) and SD2. Since the core body temperature estimation model includes SD2, the core body temperature can be calculated based on the heart rate variability.

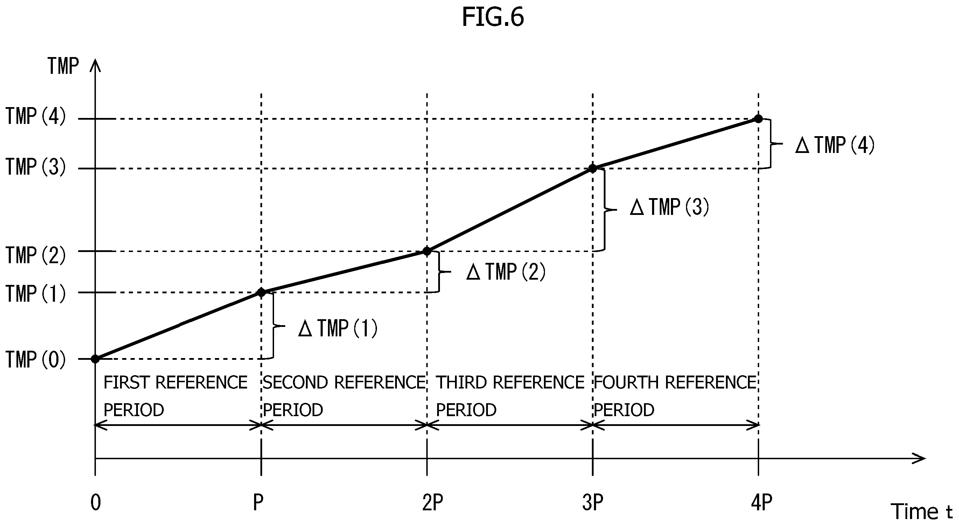

Fig. 6 is a figure illustrating an example of the relationship between the estimated value TMP of the core body temperature and the change amount $\Delta$TMP of the core body temperature. In the graph in Fig. 6, the horizontal axis represents time t and the vertical axis represents the estimated value TMP of the core body temperature. Where, TMP(0) is the measured value of the core body temperature at the initial time. Each $\Delta$TMP(j) can be estimated for each of the reference periods. For example, the change amount of the core body temperature in the second reference period $\Delta$TMP(2) is the change amount of the core body temperature from the time (t = P) at the end of the first reference period to the time (t = 2P) at the end of the second reference period. In addition, for example, the estimated value TMP(2) of the core body temperature at the end of the second reference period can be represented as TMP(1) + $\Delta$TMP(2) = TMP(0) + $\Delta$TMP(1) + $\Delta$TMP(2). The time (t = nP) at the end of the n-th reference period is the same as the time (t = (n + 1)P - P = nP) at the start of the (n + 1)-th reference period.

The estimation model building unit 106 may use other physiological indexes as the explanatory variables in building the core body temperature estimation model. Other physiological indexes may be, for example, the gender, the age, the skin temperature, the blood pressure, the exhaled gas, the pulse wave, the blood oxygen concentration, the blood flow amount, the exercise volume, and the respiratory rate. Furthermore, the temperature inside clothes, the wet bulb globe temperature (WBGT), meteorological data, and the like may be added to the explanatory variables. More accurate estimation can be performed by building a core body temperature estimation model in consideration of these explanatory variables.

<Estimation of the core body temperature>

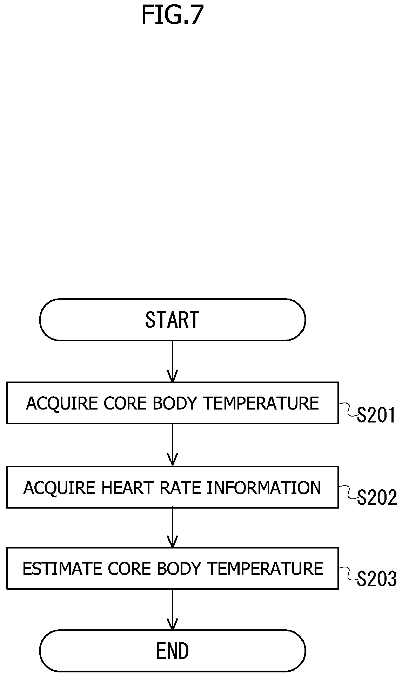

Fig. 7 is a figure illustrating an example of an operation flow of core body temperature estimation by the core body temperature estimation device. Here, the core body temperature estimation device 200 acquires the core body temperature at the initial time (first time) and the heart rate information in the period including the first time of the estimation target subject, and estimates the core body temperature of the estimation target subject using the core body temperature estimation model built by the core body temperature estimation model building device 100 based on the operation flow in Fig. 4. The heart rate information of the estimation target subject is measured by an electrocardiograph or the like. In addition, the core body temperature of the estimation target subject is measured with a thermometer or the like at the initial time (first time). The core body temperature of the estimation target subject is estimated based on the core body temperature at the initial time and the heart rate information in the period including the initial time. The core body temperature of the estimation target subject is not measured after the initial time. It is assumed that the estimation target subject is performing, for example, some work.

In S201, the initial core body temperature acquisition unit 202 of the core body temperature estimation device 200 acquires the core body temperature, at the initial time, of the estimation target subject measured by a thermometer or the like for measuring the core body temperature. The initial time is the start time of the period in which the core body temperature is estimated. The initial core body temperature acquisition unit 202 acquires the core body temperature, at the initial time, of the estimation target subject from a thermometer or the like connected to the core body temperature estimation device 200 directly or via a network or the like. In addition, the initial core body temperature acquisition unit 202 may acquire the core body temperature by causing the estimation target subject, another manager, or the like to input the core body temperature with input means such as a keyboard. The initial core body temperature acquisition unit 202 stores the acquired core body temperature in the storage unit 208. The initial core body temperature acquisition unit 202 acquires the core body temperature, at the initial time, of the estimation target subject and does not acquire the core body temperature after the initial time. When it is difficult to measure the core body temperature, at the initial time, of the estimation target subject, the core body temperature estimated based on the eardrum temperature or the skin surface temperature, at the initial time, of the estimation target subject may be used as the core body temperature at the initial time.

In S202, the heart rate information acquisition unit 204 acquires the heart rate information of the estimation target subject measured by an electrocardiograph or the like for measuring the heart rate information. The electrocardiograph may be similar to the electrocardiograph used in S102 in Fig. 4. The heart rate information acquisition unit 204 acquires the heart rate information of the estimation target subject from the thermometer or the like connected to the core body temperature estimation device 200 directly or via a network or the like. Alternatively, the heart rate information acquisition unit 204 may acquire the heart rate information from other information processing devices that have acquired the heart rate information from the electrocardiograph or the like via a network or the like. The heart rate information acquisition unit 204 acquires the heart rate information of the period including the initial time. The heart rate information acquisition unit 204 acquires the heart rate information, for example, from the time one reference period before the initial time to an arbitrary time after the initial time. The reference period is, for example, three minutes. The reference period here is the same as the reference period when the core body temperature estimation model is built. The heart rate information acquisition unit 204 stores the acquired heart rate information of the estimation target subject in the storage unit 208.

The core body temperature and the heart rate information are stored in the storage unit 208 together with the time information indicating the measured time.

In S203, the estimation unit 206 estimates the core body temperature using the core body temperature estimation model built by the operation flow in Fig. 4 based on the acquired core body temperature at the initial time and the acquired heart rate information after the initial time of the estimation target subject. The estimation unit 206 can estimate the core body temperature by adding the change amount of the core body temperature in each of the reference periods to the core body temperature at the initial time (first time) using the change amount of the core body temperature estimated by the core body temperature estimation model.

The estimation unit 206 extracts the core body temperature estimation model stored in the storage unit 208. In addition, the estimation unit 206 calculates the RRI of each heart rate based on the acquired heart rate information. The estimation unit 206 divides the calculated RRI of each heart rate for each of the reference periods (for example, every three minutes) from the time one reference period before the initial time and creates a Poincaré plot for each of the reference periods. The estimation unit 206 calculates the index SD2 for each of the reference periods based on the Poincaré plot of RRI. The estimation unit 206 the change amount ($\Delta$TMP) of the core body temperature in each of the reference periods using the core body temperature estimation model based on the core body temperature at the initial time (arbitrary time) of the estimation target subject and the index SD2 for each of the reference periods. In addition, the estimation unit 206 estimates the core body temperature (TMP) in each of the reference periods (end time of each of the reference periods) of the estimation target subject by adding the change amount of the core body temperature in each of the reference periods to the core body temperature at the initial time. The estimated core body temperature and the like are stored in the storage unit 208.

In addition, the estimation unit 206 may warn of the risk of heatstroke or the like when the estimated core body temperature becomes a predetermined threshold or higher. The estimation unit 206 may output a warning of the risk of heatstroke or the like by using output means such as a display or a speaker. This enables the estimation target subject or the like to grasp the risk of heatstroke and the like. The estimation unit 206 may simply output the estimated core body temperature using the output means. This enables the estimation target subject or the like to grasp the estimated value of the core body temperature based on the heart rate information.

The estimation unit 206 may estimate the core body temperature for each of the reference periods by calculating SD2 based on the RRI. Furthermore, the estimation unit 206 may output the core body temperature estimated for each of the reference periods using the output means. This enables the estimation target subject or the like to grasp the core body temperature in real time by the output means of the core body temperature estimation device 200.

The core body temperature estimation device 200 may be integrated with the core body temperature estimation model building device 100 that builds the core body temperature estimation model of the operation flow in Fig. 4, in advance. In addition, the core body temperature estimation device 200 may include the electrocardiograph for measuring the heart rate information of the estimation target subject. In this case, the heart rate information acquisition unit 204 of the core body temperature estimation device 200 acquires the heart rate information from this electrocardiograph.

In the core body temperature estimation device 200, the calculation of $\Delta$TMP(1) or the like requires SD2(0), which is calculated based on the heart rate information from the time (t = -P) time P before the initial time to the initial time (time t = 0). For example, the core body temperature TMP(1) at time t = P is measured with a thermometer or the like (or the core body temperature TMP(1) at time t = P is estimated based on the eardrum temperature and the skin surface temperature at time t = P) and acquired, and then $\Delta$TMP(j) and TMP(j) after the time t = 2 × P (j = 2) may be calculated. In addition, by considering the core body temperature TMP(1) at time t = P to be the same as the core body temperature TMP(0) at time t = 0, TMP(1) may be the same as TMP(0). That is, the heart rate information from time t = -P to time t = 0 is not necessary.

(Others)

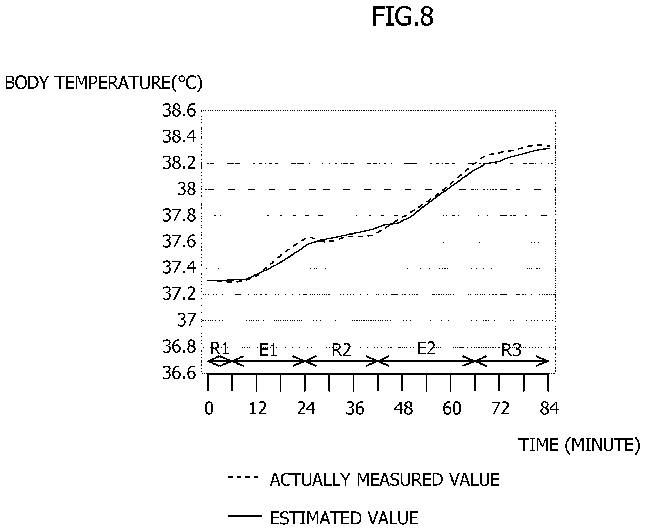

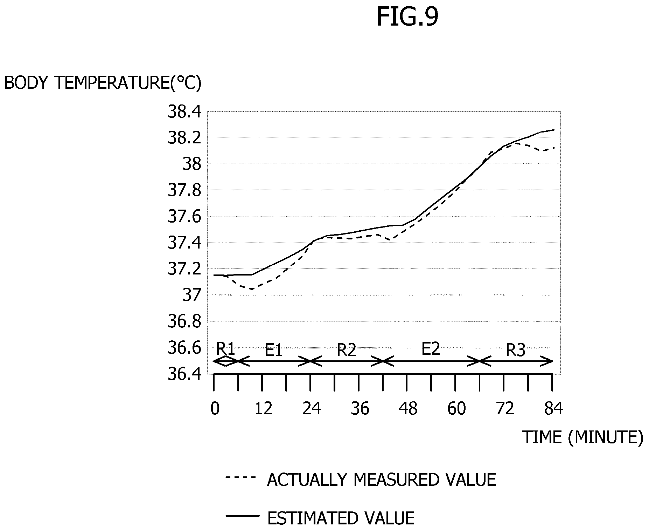

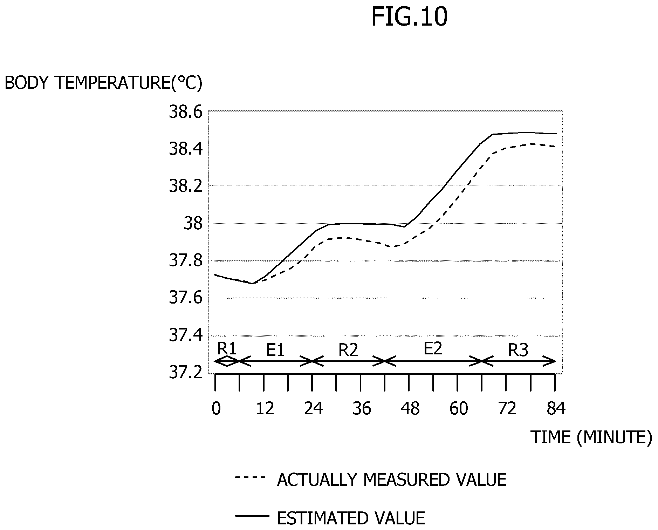

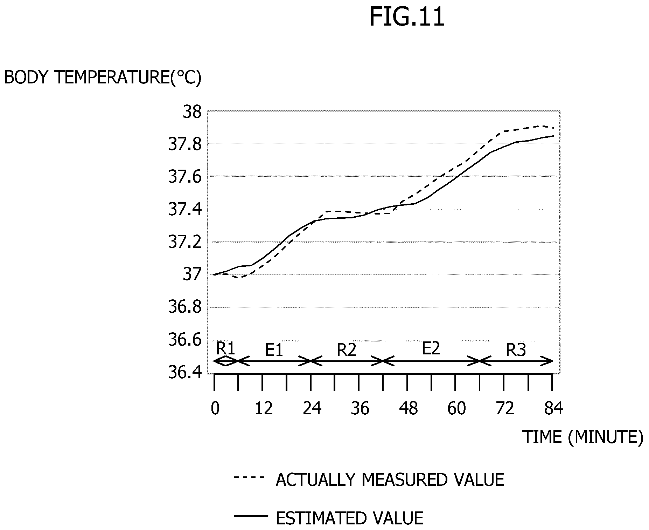

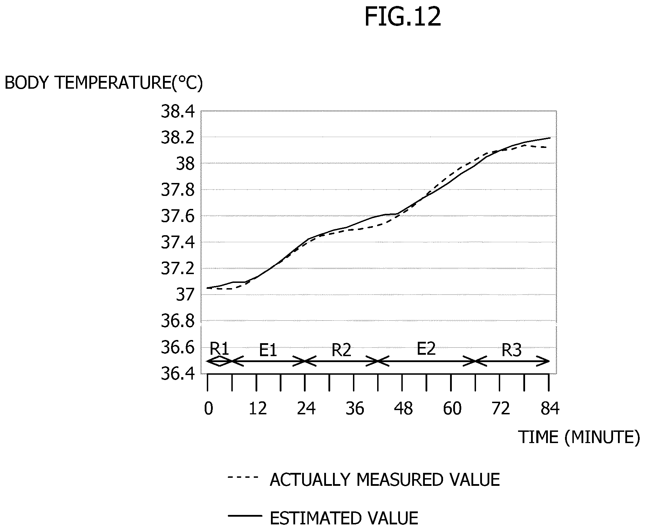

Figs. 8 to 12 are graphs illustrating examples of changes in the core body temperatures actually measured during exercise stress tests and the core body temperatures estimated based on heart rate information when the exercise stress tests were performed on subjects A to E, respectively. In each of the graphs in Figs. 8 to 12, the horizontal axis represents the time from the start of the exercise stress test and the vertical axis represents the core body temperature. In each of the graphs in Figs. 8 to 12, the dotted line represents the actually measured core body temperature and the solid line represents the estimated core body temperature. At the time of the exercise stress test, each of the subjects performs predetermined stress work under a predetermined environment (such as at a predetermined temperature and a predetermined humidity) from the start time to the end time of a predetermined period. Here, the exercise stress test is performed as follows: each of the subjects keeps quiet (take a rest) for six minutes (R1), suffers exercise stress for 18 minutes (E1), takes a rest for 18 minutes (R2), suffers exercise stress for 24 minutes (E2), and takes a rest for 18 minutes (R3) in an artificial weather room in which the temperature is adjusted to 35°C and the humidity is adjusted to 50% when the subject suffers exercise stress or takes a rest. The exercise stress applied by an ergometer is 80 kW or 60% of the maximal oxygen uptake of each of the subjects measured in advance. During the exercise stress test, the core body temperature and the heart rate information of each of the subject are continuously measured in parallel by a thermometer or the like and an electrocardiograph or the like. In estimating the core body temperature, the core body temperature of the subject that has been actually measured at the start of the exercise stress test is used. In the graphs in Figs. 8 to 12, the estimated core body temperatures of the subjects generally match the actually measured core body temperatures of the subjects. The core body temperatures of the subjects can be estimated by estimation method described above.

(Operation and effect of the embodiment)

The core body temperature estimation model building device 100 acquires the core body temperatures (such as, for example, the rectal temperatures) and the heart rate information (such as, for example, the electrocardiogram data) of the plurality of model building subjects after the initial time. The core body temperature estimation model building device 100 calculates the RRI (or other beat intervals) based on the heart rate information and creates a Poincaré plot of the RRI for each of the reference periods. The core body temperature estimation model building device 100 calculates SD2 for each of the reference periods based on the RRI. The core body temperature estimation model building device 100 performs a regression analysis that uses SD2(j) and SD2(j - 1) acquired from the analysis of the Poincaré plot of the RRI and the estimated value TMP(j - 1) of the estimated core body temperature as the explanatory variables and the estimated change amount $\Delta$TMP(j) of the core body temperature as the objective variable and builds a core body temperature estimation model. The estimated value TMP(j) of the core body temperature at time t = jP is expressed as TMP(j - 1) + $\Delta$TMP(j). In addition, TMP(0) is the core body temperature at the initial time. The core body temperature estimation model building device 100 can build a core body temperature estimation model that estimates the core body temperature based on the core body temperature and the heart rate information of the model building subject after the initial time.

The core body temperature estimation device 200 acquires the core body temperature of the model building subject at the initial time. The core body temperature estimation device 200 acquires the heart rate information in the period including the initial time of the estimation target subject. The core body temperature estimation device 200 estimates the core body temperature of the estimation target subject based on the core body temperature at the initial time and the heart rate information in the period including the initial time using the core body temperature estimation model. The core body temperature estimation device 200 can easily estimate the core body temperature, which is difficult to constantly measure during work or the like, by using a physiological index such as the heart rate information with a core body temperature estimation model.

A core body temperature estimation model has been built by the core body temperature estimation model building device 100 using the core body temperature and heart rate information measured when the model building subject has performed the exercise stress test described above. The estimated value of the core body temperature has been calculated by the core body temperature estimation device 200 based on the built core body temperature estimation model using the core body temperature at the initial time and the measured heart rate information. The error between the measured value of the core body temperature and the estimated value of the core body temperature based on the core body temperature estimation model was -0.007°C on average (-0.50°C at maximum) and the average error rate is -0.02% (-1.30% at maximum). The core body temperature can be estimated accurately using the core body temperature and the heart rate information at the initial time based on the core body temperature estimation model.

<Computer readable recording medium>

A program that enables a computer or other machine or device (referred to below as a computer etc.) to achieve any of the above functions can be recorded in a computer etc. readable recording medium. Then, the function can be provided by causing the computer etc. to read and execute the program in this recording medium.

Here, a computer etc. readable recording medium is a recording medium that can store information such as data and programs by electrical, magnetic, optical, mechanical, or chemical action and can be read by a computer etc. Such a recording medium may be provided with components constituting a computer, such as a CPU and a memory so that the CPU executes programs.

In addition, such recording media that can be removed from a computer etc. include, for example, a flexible disk, a magneto-optical disk, a CD-ROM, a CD-R/W, a DVD, a DAT, an 8-mm tape, a memory card, and the like.

In addition, recording media that are fixed to a computer etc. include a hard disk drive, a ROM, an SSD, and the like.

Reference Signs List

| | |
|---|---|
| 100 | Core body temperature estimation model building device |
| 102 | Core body temperature acquisition unit |
| 104 | Heart rate information acquisition unit |
| 106 | Estimation model building unit |
| 108 | Storage unit |
| 200 | Core body temperature estimation device |
| 202 | Initial core body temperature acquisition unit |
| 204 | Heart rate information acquisition unit |
| 206 | Estimation unit |
| 208 | Storage unit |

**Claims**

1. A core body temperature estimation device (200) comprising:

   a core body temperature acquisition unit (202) that acquires a core body temperature, at a first time, of an estimation target subject targeted for core body temperature estimation;
   a beat acquisition unit (204) that acquires a beat, in a period including the first time, of the estimation target subject; and
   an estimation unit (206) that estimates the core body temperature of the estimation target subject based on the core body temperature at the first time acquired by the core body temperature acquisition unit (202) and the beat in the period including the first time acquired by the beat acquisition unit (204), by using a core body temperature estimation model that estimates a core body temperature at a predetermined time based on a core body temperature at an initial time and a beat from the initial time to the predetermined time,
   **characterized in that**
   the core body temperature estimation model estimates a change amount of the core body temperature for each of reference periods each having a predetermined time length and is built by a regression analysis that uses, as explanatory variables, an index SD2(n) of an n-th reference period, an index SD2(n-1) of an (n-1)-th reference period and the estimated core body temperatures of model building subjects at a start of the n-th reference period and uses, as an objective variable, change amounts of the core body temperatures in the n-th reference period of the model building subjects, the index SD2(n) and the index SD2(n-1) are an index based on a distance from each point in the reference period to a gravity center line that passes through a center of gravity of all points in the reference period and is orthogonal to the identity line that the coordinate on the horizontal axis is identical to the coordinate on the vertical axis in a Poincaré plot of beat intervals that are intervals of beats of a plurality of model building subjects
   wherein the estimation unit (206) estimates the core body temperature after the first time of the estimation target subject by adding the change amount of the core body temperature estimated for each of the reference periods after the first time to the core body temperature at the first time.

2. The core body temperature estimation device according to claim 1,
   wherein the index reflects heart rate variability.

3. The core body temperature estimation device according to any one of claims 1 to 2, further comprising a pulsometer including an electrode for measuring a beat of the estimation target subject,
   wherein the beat acquisition unit (204) acquires the beat of the estimation target subject measured by the pulsometer.

4. A core body temperature estimation method executed by a computer, the method comprising:

   acquiring a core body temperature, at a first time, of an estimation target subject targeted for core body temperature estimation;
   acquiring a beat, in a period including the first time, of the estimation target subject; and
   estimating the core body temperature of the estimation target subject, based on the core body temperature at the first time and the beat in the period including the first time by using a core body temperature estimation model that estimates a core body temperature at a predetermined time, based on a core body temperature at an initial time and a beat from the initial time to the predetermined time,
   wherein the core body temperature estimation model estimates a change amount of the core body temperature for each of reference periods each having a predetermined time length and is built by a regression analysis that uses, as explanatory variables, an index SD2(n) of an n-th reference period, an index SD2(n-1) of an (n-1)-th reference period and the estimated core body temperatures of model building subjects at a start of the n-th reference period and uses, as an objective variable, change amounts of the core body temperatures in the n-th reference period of the model building subjects, the index SD2(n) and the index SD2(n-1) are an index based on a distance from each point in the reference period to a gravity center line that passes through a center of gravity of all points in the reference period and is orthogonal to the identity line that the coordinate on the horizontal axis is identical to the coordinate on the vertical axis in a Poincaré plot of beat intervals that are intervals of beats of a plurality of model building subjects
   wherein the estimation unit estimates the core body temperature after the first time of the estimation target subject by adding the change amount of the core body temperature estimated for each of the reference periods after the first time to the core body temperature at the first time.

5. A core body temperature estimation program executed by a computer, the program implementing the method of claim 4.

**Patentansprüche**

1. Vorrichtung (200) zur Schätzung einer Körperkerntemperatur, umfassend:

   eine Körperkerntemperatur-Erfassungseinheit (202), die eine Körperkerntemperatur einer Schätzungszielperson, auf deren Körperkerntemperaturschätzung abgezielt wird, zu einem ersten Zeitpunkt erfasst;
   eine Herzschlag-Erfassungseinheit (204), die einen Herzschlag der Schätzungszielperson in einer Periode erfasst, die den ersten Zeitpunkt umfasst; und
   eine Schätzungseinheit (206), welche die Körperkerntemperatur der Schätzungszielperson auf der Grundlage der zum ersten Zeitpunkt von der Körperkerntemperatur-Erfassungseinheit (202) erfassten Körperkerntemperatur und des in der den ersten Zeitpunkt umfassenden Periode durch die Herzschlag-Erfassungseinheit (204) erfassten Herzschlags unter Verwendung eines Körperkerntemperatur-Schätzungsmodells schätzt, das eine Körperkerntemperatur zu einem vorbestimmten Zeitpunkt auf der Grundlage einer Körperkerntemperatur zu einem Anfangszeitpunkt und eines Herzschlags von dem Anfangszeitpunkt bis zum vorbestimmten Zeitpunkt schätzt,
   **dadurch gekennzeichnet, dass**
   das Körperkerntemperatur-Schätzungsmodell einen Änderungsbetrag der Körperkerntemperatur für jede von Bezugsperioden schätzt, die jeweils eine vorbestimmte Zeitdauer aufweisen, und durch eine Regressionsanalyse aufgebaut wird, die als erklärende Variablen einen Index SD2(n) einer n-ten Bezugsperiode, einen Index SD2(n-1) einer (n-1)-ten Bezugsperiode und die geschätzten Körperkerntemperaturen von Modellaufbaupersonen zu einem Beginn der n-ten Bezugsperiode verwendet und als eine Zielvariable Änderungsbeträge der Körperkerntemperaturen in der n-ten Bezugsperiode der Modellaufbaupersonen verwendet, wobei der Index SD2(n) und der Index SD2(n-1) ein Index sind, der auf einem Abstand von jedem Punkt in der Bezugsperiode zu einer Schwerpunktmittellinie gründet, die einen Schwerpunkt aller Punkte in der Bezugsperiode durchquert und orthogonal zur Identitätslinie ist, auf der die Koordinate auf der horizontalen Achse identisch mit der Koordinate auf der vertikalen Achse in einem Poincaré-Plot von Herzschlagintervallen ist, die Intervalle von Herzschlägen einer Vielzahl von Modellaufbaupersonen sind,

wobei die Schätzungseinheit (206) die Körperkerntemperatur der Schätzungszielperson nach dem ersten Zeitpunkt durch Addieren des Änderungsbetrags der für jede der Bezugsperioden nach dem ersten Zeitpunkt geschätzten Körperkerntemperatur zur Körperkerntemperatur zum ersten Zeitpunkt schätzt.

2. Körperkerntemperatur-Schätzungsvorrichtung nach Anspruch 1,
wobei der Index die Herzschlagfrequenzveränderlichkeit widerspiegelt.

3. Körperkerntemperatur-Schätzungsvorrichtung nach einem der Ansprüche 1 bis 2, ferner umfassend ein Pulsometer, das eine Elektrode zum Messen eines Herzschlags der Schätzungszielperson umfasst,
wobei die Herzschlag-Erfassungseinheit (204) den von dem Pulsometer gemessenen Herzschlag der Schätzungszielperson erfasst.

4. Körperkerntemperatur-Schätzungsverfahren, das von einem Computer ausgeführt wird, wobei das Verfahren umfasst:

Erfassen einer Körperkerntemperatur einer Schätzungszielperson, auf deren Körperkerntemperaturschätzung abgezielt wird, zu einem ersten Zeitpunkt;
Erfassen eines Herzschlags der Schätzungszielperson in einer Periode, die den ersten Zeitpunkt umfasst; und
Schätzen der Körperkerntemperatur der Schätzungszielperson auf der Grundlage der Körperkerntemperatur zum ersten Zeitpunkt und des Herzschlags in der Periode, die den ersten Zeitpunkt umfasst, unter Verwendung eines Körperkerntemperatur-Schätzungsmodells, das eine Körperkerntemperatur zu einem vorbestimmten Zeitpunkt auf der Grundlage einer Körperkerntemperatur zu einem Anfangszeitpunkt und eines Herzschlags von dem Anfangszeitpunkt bis zum vorbestimmten Zeitpunkt schätzt,
wobei das Körperkerntemperatur-Schätzungsmodell einen Änderungsbetrag der Körperkerntemperatur für jede von Bezugsperioden schätzt, die jeweils eine vorbestimmte Zeitdauer aufweisen, und durch eine Regressionsanalyse aufgebaut wird, die als erklärende Variablen einen Index SD2(n) einer n-ten Bezugsperiode, einen Index SD2(n-1) einer (n-1)-ten Bezugsperiode und die geschätzten Körperkerntemperaturen von Modellaufbaupersonen zu einem Beginn der n-ten Bezugsperiode verwendet und als eine Zielvariable Änderungsbeträge der Körperkerntemperaturen in der n-ten Bezugsperiode der Modellaufbaupersonen verwendet, wobei der Index SD2(n) und der Index SD2(n-1) ein Index sind, der auf einem Abstand von jedem Punkt in der Bezugsperiode zu einer Schwerpunktmittellinie gründet, die einen Schwerpunkt aller Punkte in der Bezugsperiode durchquert und orthogonal zur Identitätslinie ist, auf der die Koordinate auf der horizontalen Achse identisch mit der Koordinate auf der vertikalen Achse in einem Poincaré-Plot von Herzschlagintervallen ist, die Intervalle von Herzschlägen einer Vielzahl von Modellaufbaupersonen sind,
wobei die Schätzungseinheit die Körperkerntemperatur der Schätzungszielperson nach dem ersten Zeitpunkt durch Addieren des Änderungsbetrags der für jede der Bezugsperioden nach dem ersten Zeitpunkt geschätzten Körperkerntemperatur zur Körperkerntemperatur zum ersten Zeitpunkt schätzt.

5. Körperkerntemperatur-Schätzungsprogramm, das von einem Computer ausgeführt wird, wobei das Programm das Verfahren nach Anspruch 4 implementiert.

**Revendications**

1. Dispositif d'estimation de température corporelle centrale (200), comprenant :

une unité d'acquisition de température corporelle centrale (202) qui acquiert une température corporelle centrale, à un premier instant, d'un sujet cible d'estimation ciblé pour une estimation de température corporelle centrale ;
une unité d'acquisition de pouls (204) qui acquiert un pouls, dans une période incluant le premier instant, du sujet cible d'estimation ; et
une unité d'estimation (206) qui estime la température corporelle centrale du sujet cible d'estimation sur la base de la température corporelle centrale au premier instant, acquise par l'unité d'acquisition de température corporelle centrale (202), et du pouls dans la période incluant le premier instant, acquis par l'unité d'acquisition de pouls (204), en utilisant un modèle d'estimation de température corporelle centrale qui estime une température corporelle centrale à un instant prédéterminé sur la base de la température corporelle centrale à un instant initial et du pouls entre l'instant initial et l'instant prédéterminé,

**caractérisé en ce que**

le modèle d'estimation de température corporelle centrale estime une ampleur de changement de la température corporelle centrale pour chacune des périodes de référence ayant chacune une longueur de temps prédéterminée et est construit au moyen d'une analyse de régression qui utilise, à titre de variables explicatives, un indice SD2(n) d'une n-ième période de référence, un indice SD2(n-1) d'une (n-1)-ième période de référence et les températures corporelles centrales estimées de sujets de construction de modèle au début de la n-ième période de référence et utilise, à titre de variable objective, les ampleurs de changement des températures corporelles centrales dans la n-ième période de référence des sujets de construction de modèle, l'indice SD2(n) et l'indice SD2 (n-1) sont des indices basés sur la distance allant de chaque point dans la période de référence à la ligne centrale de gravité qui passe par le centre de gravité de tous les points dans la période de référence et est orthogonale à la ligne d'identité pour laquelle la coordonnée sur l'axe horizontal est identique à la coordonnée sur l'axe vertical dans un tracé de Poincaré d'intervalles entre battements qui sont des intervalles entre battements d'une pluralité de sujets de construction de modèle,

dans lequel l'unité d'estimation (206) estime la température corporelle centrale après le premier instant du sujet cible d'estimation en ajoutant l'ampleur de changement de la température corporelle centrale estimée pour chacune des périodes de référence après le premier instant à la température corporelle centrale au premier instant.

2.  Dispositif d'estimation de température corporelle centrale selon la revendication 1, dans lequel l'indice reflète la variabilité de la fréquence cardiaque.

3.  Dispositif d'estimation de température corporelle centrale selon l'une quelconque des revendications 1 et 2, comprenant en outre un pulsomètre incluant une électrode pour mesurer le pouls du sujet cible d'estimation, dans lequel l'unité d'acquisition de pouls (204) acquiert le pouls du sujet cible d'estimation mesuré par le pulsomètre.

4.  Méthode d'estimation de température corporelle centrale exécutée par un ordinateur, la méthode comprenant :

    l'acquisition d'une température corporelle centrale, à un premier instant, d'un sujet cible d'estimation ciblé pour une estimation de température corporelle centrale ;

    l'acquisition d'un pouls, dans une période incluant le premier instant, du sujet cible d'estimation ; et

    l'estimation de la température corporelle centrale du sujet cible d'estimation sur la base de la température corporelle centrale au premier instant et du pouls dans la période incluant le premier instant, par utilisation d'un modèle d'estimation de température corporelle centrale qui estime une température corporelle centrale à un instant prédéterminé sur la base de la température corporelle centrale à un instant initial et du pouls entre l'instant initial et l'instant prédéterminé,

    dans laquelle le modèle d'estimation de température corporelle centrale estime une ampleur de changement de la température corporelle centrale pour chacune des périodes de référence ayant chacune une longueur de temps prédéterminée et est construit au moyen d'une analyse de régression qui utilise, à titre de variables explicatives, un indice SD2(n) d'une n-ième période de référence, un indice SD2(n-1) d'une (n-1)-ième période de référence et les températures corporelles centrales estimées de sujets de construction de modèle au début de la n-ième période de référence et utilise, à titre de variable objective, les ampleurs de changement des températures corporelles centrales dans la n-ième période de référence des sujets de construction de modèle, l'indice SD2(n) et l'indice SD2 (n-1) sont des indices basés sur la distance allant de chaque point dans la période de référence à la ligne centrale de gravité qui passe par le centre de gravité de tous les points dans la période de référence et est orthogonale à la ligne d'identité pour laquelle la coordonnée sur l'axe horizontal est identique à la coordonnée sur l'axe vertical dans un tracé de Poincaré d'intervalles entre battements qui sont des intervalles entre battements d'une pluralité de sujets de construction de modèle,

    dans laquelle l'unité d'estimation estime la température corporelle centrale après le premier instant du sujet cible d'estimation en ajoutant l'ampleur de changement de la température corporelle centrale estimée pour chacune des périodes de référence après le premier instant à la température corporelle centrale au premier instant.

5.  Programme d'estimation de température corporelle centrale exécuté par un ordinateur, le programme implémentant la méthode de la revendication 4.

# FIG.1

```
┌─────────────────────────────────────────────┐
│                                              │
│   ┌──────────────────────────────────┐       │
│   │   CORE BODY TEMPERATURE          │ ⌐102  │
│   │   ACQUISITION UNIT               │       │
│   └──────────────────────────────────┘       │
│                                              │
│   ┌──────────────────────────────────┐       │
│   │   HEART RATE INFORMATION         │ ⌐104  │
│   │   ACQUISITION UNIT               │       │
│   └──────────────────────────────────┘       │
│                                              │
│   ┌──────────────────────────────────┐       │
│   │   ESTIMATION MODEL               │ ⌐106  │
│   │   BUILDING UNIT                  │       │
│   └──────────────────────────────────┘       │
│                                              │
│   ┌──────────────────────────────────┐       │
│   │   STORAGE UNIT                   │ ⌐108  │
│   └──────────────────────────────────┘       │
│                                           ⌐100│
│                                              │
│        CORE BODY TEMPERATURE                 │
│      ESTIMATION MODEL BUILDING DEVICE        │
│                                              │
└─────────────────────────────────────────────┘
```

# FIG.2

```
┌─────────────────────────────────────────┐
│                                           │
│   ┌─────────────────────────────────┐     │
│   │      INITIAL CORE BODY           │  ⌐ 202
│   │ TEMPERATURE ACQUISITION UNIT     │     │
│   └─────────────────────────────────┘     │
│                                           │
│   ┌─────────────────────────────────┐     │
│   │    HEART RATE INFORMATION        │  ⌐ 204
│   │      ACQUISITION UNIT            │     │
│   └─────────────────────────────────┘     │
│                                           │
│   ┌─────────────────────────────────┐     │
│   │       ESTIMATION UNIT            │  ⌐ 206
│   └─────────────────────────────────┘     │
│                                           │
│   ┌─────────────────────────────────┐     │
│   │        STORAGE UNIT              │  ⌐ 208
│   └─────────────────────────────────┘     │
│                                        ⌐ 200
│        CORE BODY TEMPERATURE              │
│         ESTIMATION DEVICE                 │
└─────────────────────────────────────────┘
```

# FIG.3

```
INFORMATION
PROCESSING DEVICE

91 ─ PROCESSOR ──────┬──── MEMORY ─── 92
                     │
                     ├──── STORAGE UNIT ─── 93
                     │
                     ├──── INPUT UNIT ─── 94
                     │
                     ├──── OUTPUT UNIT ─── 95
                     │
                     └──── COMMUNICATION
                           CONTROL UNIT ─── 96         ─ 90
```

# FIG.4

```
          ┌─────────────┐
          │    START    │
          └──────┬──────┘
                 │
                 ▼
   ┌────────────────────────────────┐
   │ ACQUIRE CORE BODY TEMPERATURE  │ ⌐S101
   └────────────────┬───────────────┘
                    │
                    ▼
   ┌────────────────────────────────┐
   │  ACQUIRE HEART RATE INFORMATION │ ⌐S102
   └────────────────┬───────────────┘
                    │
                    ▼
   ┌────────────────────────────────┐
   │  BUILD CORE BODY TEMPERATURE   │ ⌐S103
   │      ESTIMATION MODEL          │
   └────────────────┬───────────────┘
                    │
                    ▼
          ┌─────────────┐
          │     END     │
          └─────────────┘
```

# FIG.5

# FIG.6

FIG.7

```
                    ┌─────────────────────────┐
                    │          START          │
                    └────────────┬────────────┘
                                 │
                                 ▼
        ┌──────────────────────────────────────────┐
        │    ACQUIRE CORE BODY TEMPERATURE          │  S201
        └────────────────────┬─────────────────────┘
                             │
                             ▼
        ┌──────────────────────────────────────────┐
        │    ACQUIRE HEART RATE INFORMATION         │  S202
        └────────────────────┬─────────────────────┘
                             │
                             ▼
        ┌──────────────────────────────────────────┐
        │    ESTIMATE CORE BODY TEMPERATURE         │  S203
        └────────────────────┬─────────────────────┘
                             │
                             ▼
                    ┌─────────────────────────┐
                    │           END           │
                    └─────────────────────────┘
```

# FIG.8

BODY TEMPERATURE(°C)

TIME (MINUTE)

---- ACTUALLY MEASURED VALUE

—— ESTIMATED VALUE

EP 4 030 157 B1

# FIG.9

BODY TEMPERATURE(°C)

TIME (MINUTE)

---- ACTUALLY MEASURED VALUE

—— ESTIMATED VALUE

## FIG.10

BODY TEMPERATURE(°C)

ACTUALLY MEASURED VALUE

ESTIMATED VALUE

TIME (MINUTE)

EP 4 030 157 B1

# FIG.11

BODY TEMPERATURE(°C)

TIME (MINUTE)

---- ACTUALLY MEASURED VALUE

—— ESTIMATED VALUE

EP 4 030 157 B1

# FIG.12

BODY TEMPERATURE(°C)

EP 4 030 157 B1

---- ACTUALLY MEASURED VALUE

—— ESTIMATED VALUE

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140180027 A1
- JP 2017166075 A
- JP 2017027123 A